Europäisches Patentamt

European Patent Office

Office européen des brevets

(19)

(11) Publication number : **0 384 722 B1**

# (12) EUROPEAN PATENT SPECIFICATION

(45) Date of publication of patent specification :
08.09.93 Bulletin 93/36

(51) Int. Cl.⁵ : **A61K 31/635, // (A61K31/635, 31:505)**

(21) Application number : **90301842.2**

(22) Date of filing : **21.02.90**

(54) **Chemical compositions.**

(30) Priority : **22.02.89 GB 8903978**

(43) Date of publication of application :
**29.08.90 Bulletin 90/35**

(45) Publication of the grant of the patent :
**08.09.93 Bulletin 93/36**

(84) Designated Contracting States :
**AT BE CH DE DK ES FR GB GR IT LI LU NL SE**

(56) References cited :
**EP-A- 0 051 879**
**EP-A- 0 238 207**
**CHEMICAL ABSTRACTS, vol. 76, no. 18, 1st May 1972, page 280, astract no. 103651m, Columbus, Ohio, US; "New animal drugs in oral dosage forms. Sulfadimethoxine", & FED. REGIST. 16 Feb 1972, 37(32), 3426**

(56) References cited :
**UNLISTED DRUGS, vol. 38, July 1986 (CHATHAM, New Jersey, U.S.A.)**
**J. vet. Pharmacol. Therap., 5, 131-135, 1982; Riffat et al.**
**Zbl. Vet. Med. A, 27, 75-80 (1980); M. Nawaz**
**Can. J. comp. Med., 40, 310-317 (1976); J.D. Baggot et al.**

(73) Proprietor : **PITMAN-MOORE LIMITED**
**Breakspear Road South Harefield**
**Uxbridge Middlesex UB9 6LS (GB)**

(72) Inventor : **White, Geoffrey, Coopers Animal Health Limited**
**Berkhamsted Hill, Berkhamsted**
**Hertfordshire (GB)**

(74) Representative : **Matthews, Derek Peter et al**
**Frank B. Dehn & Co. Imperial House 15-19 Kingsway**
**London WC2B 6UZ (GB)**

EP 0 384 722 B1

EP 0 384 722 B1

## Description

The present invention relates to synergistic compositions of a sulphonamide and a 5- arylmethyl-2,4-diaminopyrimidine and their use in the treatment of microbial infections, particularly in domestic animals.

European Patent Application 51879 discloses a number of compounds including 2,4-diamino-5-(8-dimethylamino-7-methyl-5-quinolylmethyl) pyrimidine (which now has the approved name Baquiloprim) as having good _in vitro_ antibacterial activity against a range of organisms.

European Patent Application No. 238207 describes combinations of Baquiloprim with sulphadimidine for the treatment of bacterial infections in livestock. However such combinations are generally unsuited to the treatment of cats and dogs due to the unsuitable pharmacokinetic properties of sulphadimidine in these species.

It has now suprisingly been discovered that baquiloprim has a significantly greater half-life in dogs than in other species and it has further been found that its combination with sulphadimethoxine provides a synergistic composition suitable for the effective treatment of pathogenic bacteria. This composition, in which the pharmacokinetic properties of the pyrimidine and sulphadimethoxine are well matched, has the advantage of administration in a low-dosage regime suitable for domestic animals.

Accordingly, the present invention provides a composition containing 2,4-diamino-5-(8-dimethylamino-7-methyl-5-quinolylmethyl)pyrimidine (baquiloprim) and sulphadimethoxine or salts thereof and optionally a pharmaceutically acceptable carrier therefor.

The compositions may be adapted for administration by routes well known to those skilled in the veterinary and formulation sciences. The pyrimidine and sulphadimethoxine may be present in the compositions as salts or in free unsalted form. It is often convenient for one of the pyrimidine and sulphadimethoxine to be present as a salt, for example sodium sulphadimethoxine or an acid addition salt of the pyrimidine, for example formed from lactic, citric or formic acids, whilst the other component is present in free unsalted form (see for example UK patents 1176395, 1347472 and 1469521). It is often convenient for the sulphadimethoxine to be present in both the salt and the free unsalted form. The compositions may be adapted for administration by any suitable route, in particular for oral, parenteral, topical or intravaginal administration.

Compositions suitable for oral administration include drenches (solutions or suspensions for oral liquid dosing by syringe) tablets, capsules or pastes. Preferred orally administrable compositions include tablets.

Alternatively, the compositions may be adapted to be administered parenterally by sub-cutaneous, intramuscular or intravenous injection of a sterile solution or suspension. Preferably parenteral administration is by subcutaneous injection.

Compositions suitable for topical administration include creams, ointments or sprays applied to the skin, and intravaginal compositions include pessaries, creams and foams.

If desired the active compounds may be presented as the raw chemicals, but preferably they are formulated with pharmaceutically acceptable carriers.

The pharmaceutically acceptable carriers present in the compositions of the present invention are materials recommended for the purpose of administering the medicament. These may be liquid, solid or gaseous materials, which are otherwise inert or medically acceptable and are compatible with the active ingredient. It will be appreciated that reference herein to pharmaceutically acceptable carriers includes carriers acceptable in the veterinary art.

For oral administration, fine powders or granules conveniently contain or are admixed with diluting agents, for example lactose, calcium carbonate, calcium phosphate or mineral carriers, dispersing agents and/or surface active agents, for example sorbitan derivatives such as Tweens or Spans, and may be presented in a drench, in water or in a syrup, in a paste, in capsules or sachets in the dry state or in a non-aqueous suspension, or in a suspension in water or syrup. Where desirable or necessary, preserving, suspending, thickening, emulsifying, colouring or flavouring agents can be included.

Tablets for oral administration are conveniently compressed from the composition which may contain disintegrating agents such as maize starch or calcium or sodium methyl celluloses, hydroxypropylmethyl cellulose, sodium alginates or sodium starch glycolates; granulating or binding agents such as starch in the form of mucilage, starch derivatives, such as "Snow Flake", cellulose derivatives such as methyl cellulose, guar based vegetable gums, gelatin or polyvinylpyrrolidone; and/or lubricating agents, such as magnesium stearate, talc, calcium stearate or stearic acid. One composition found to be particularly advantageous for the treatment of bacterial infection by oral administration comprises a tablet containing active ingredients pyrimidine: sulphonamide in the ratio 1:5 in microcrystalline cellulose. Preferably the microcrystalline cellulose constitutes 30% of the composition.

If desired the tablets may be coated using conventional coating agents. Coatings for tablets found to be particularly effective conveniently comprise Methocels (Colorcon) and polyethylene glycol.

For parenteral administration, the compounds may be presented in sterile injection solutions which may

2

contain antioxidants or buffers, or as sterile injectable suspensions. The solutions or suspensions conveniently contain solubilising, wetting and stabilizing agents, for example Tweens, PVP, surfactants and preservatives. Suitable solvents include water and organic solvents such as dimethylformamide dimethylacetamide, diethylacetamide, ethyl lactate, ethyl oleate, dimethylsulphoxide, alcohols, e.g. ethanol, and isopropanol, glycols, e.g. ethylene glycol, diethylene glycol, propylene glycol, butylene glycol and hexamethylene glycol, polyethylene glycols containing 2 to 159 ethylene glycol monomer units and having average molecular weights from about 90 to 7500, glycerol formal, glycerol, glycofurol, isopropylmyristate, N-methylpyrrolidone, Tetraglycol, tetrahydrofurfurylalcohol, 2-pyrrolidone, and fixed and neutral oils, for example fractionated coconut oil. Formulations found to be particularly advantageous for the treatment of bacterial diseases by subcutaneous injection comprise 10.8% or preferably 12% 1:5 (pyrimidine:sulphonamide) solution in Tetraglycol (Agrar special grade). These formulations have the advantage that they cause less pain at the site of injection.

Other carriers or excipients which may be included in the formulations are, for example, medically inert ingredients, e.g. solid and liquid diluents such as lactose, glucose, microcrystalline cellulose, starch or calcium phosphate for tablets, or capsules; olive oil or ethyl oleate for soft capsules; and water or vegetable oil for suspensions or emulsions; lubricating agents such as talc or magnesium stearate; gelling agents such as colloidal clays; thickening agents such as gum tragacanth or sodium alginate; dedusting agents such as liquid paraffin, fixed oils and surfactants and other therapeutically acceptable accessory ingredients such as humectants, preservatives, buffers, and antioxidants which are useful as carriers in such formulations.

When desired, other medicaments and/or nutrients such as vitamins, may also be included.

The compositions of the present invention can be prepared by the admixture of the active compounds with a pharmaceutically acceptable carrier. Other ingredients, for example the conventional pharmaceutical excipients described above may be added as required.

In view of the good oral bioavailability of the combination of the invention, the oral route of administration is a preferred one.

The doses and ratios of the components of the composition are chosen such that there is provided at the site of infection a concentration of each component sufficient to exert a synergistic effect. In general the active ingredients are present in a ratio of between 1:1 and 1:20 pyrimidine:sulphonamide. Suitably, combinations for parenteral use are those having the ratio of 1:5 pyrimidine:sulphonamide. For oral administration suitably the active ingredients are present in a ratio of between 1:4 and 1:10 pyrimidine:sulphonamide, preferably 1:5.

Administration of a composition as hereinbefore defined can be used to treat bacterial infections in animals, particularly those caused by aerobic Gram positive and Gram negative bacteria.

Examples of bacterial infections include pneumonia and upper respiratory infections, skin infections, wound infections, enteritis including salmonellosis and urinary tract infections. In general the dosages are such that the amount of the pyrimidine administered per day is in the range 0.5 mg/kg bodyweight to 10.0 mg/kg, suitably 1.0 mg/kg to 10mg/kg and preferably 1.0 mg/kg to 8 mg/kg and the amount of sulphonamide administered per day is in the range 1 mg/kg bodyweight to 100 mg/kg bodyweight, preferably 5 mg/kg bodyweight to 40 mg/kg bodyweight, although the optimal dosages will vary according to the route of administration and the species. Thus when administered to dogs by means of a tablet composition, a once in 2 days tablet dosage typically administers active ingredients in a ratio of 1:5 pyrimidine: sulphonamide at an active content in the tablet of 600mg of 60 mg, the smaller dose being suitable for administration to cats on a once daily basis.

Thus, a once-in-two-days tablet formulation for oral administration to dogs typically contains 10mg to 200mg of the pyrimidine, preferably 100mg; and 50mg to 999.9mg of sulphadimethoxine, preferably 500mg of sulphadimethoxine.

Tablet formulations for cats will normally contain respectively a unit dose of 2mg to 20mg of pyrimidine, for example 10mg of pyrimidine and 10mg to 100mg of sulphadimethoxine, for example 50mg sulphadimethoxine.

A once in 3 days injectable composition for administration to dogs, for example by subcutaneous or intramuscular injection, typically provides the combined active ingredients at dosages of 10-600 mg/kg and preferably 30mg/kg. The ratio of pyrimidine:sulphonamide is 1:5. The composition is typically adminstered up to 5 times and preferably only once.

The compositions of the present invention are particularly advantageous in the treatment of domestic species for example cats and dogs.

The invention will now be illustrated in greater detail by means of examples. In the following examples 2,4-diamino-5-(8-dimethylamino-7-methyl-5-quinolylmethyl) pyrimidine is referred to as Compound A, sulphadimethoxine is referred to a SDM, and trimethoprim as TMP.

Example 1

Bioavailability in dogs of an oral formulation of Compound A and Sulphadimethoxine

Materials and Methods

Four laboratory-bred beagles, about 9 months old were each given a single oral dose of the powdered compounds contained in a gelatine capsule, giving 5 mg/kg of Compound A and 25mg/kg of sulphadimethoxine (SDM). Bodyweights and doses are shown in Table 1.

Blood samples were collected at the times shown in Table 2, aliquots of sera being stored at -20° pending assay for Compound A by bio-assay using Bacillus pumilus as test organism and for SDM by the Bratton-Marshall method.

Table 1    Bodyweights and doses.

| Dog | Sex | Weight (kg) | Compound A (mg) | SDM (mg) |
|---|---|---|---|---|
| 1 | F | 8.8 | 44 | 220 |
| 2 | F | 10.2 | 51 | 255 |
| 4 | M | 13.2 | 66 | 330 |
| 3 | M | 15.4 | 77 | 385 |

Table 2    Serum concentrations (mg/L) of Compound A and SDM after a single oral dose at 5mg/kg and 25mg/kg respectively.

| Time (hours) | Dog 1 Compound A | SDM | Dog 2 Compound A | SDM | Dog 3 Compound A | SDM | Dog 4 Compound A | SDM |
|---|---|---|---|---|---|---|---|---|
| 0 | <0.06 | 0 | <0.06 | 0 | <0.06 | 0 | <0.06 | 0 |
| 1 | 0.44 | 46 | 0.08 | 4 | 0.21 | 9 | 0.12 | 20 |
| 4 | 0.94 | 92 | 0.82 | 91 | 0.90 | 71 | 0.92 | 79 |
| 7 | 0.80 | 96 | 0.68 | 76 | 0.94 | 61 | 0.92 | 66 |
| 24 | 0.38 | 50 | 0.18 | 28 | 0.29 | 34 | 0.30 | 36 |
| 27 | 0.30 | 49 | 0.29 | 29 | 0.25 | 26 | 0.27 | 28 |
| 30 | 0.30 | 42 | 0.23 | 25 | 0.19 | 19 | 0.12 | 24 |
| 48 | 0.16 | 28 | 0.13 | 23 | 0.08 | 8 | 0.07 | 11 |
| 51 | <0.06 | 25 | 0.11 | 22 | <0.06 | 4 | 0.08 | 10 |

Results and Discussion

High serum concentrations of both compounds (see Table 2) were achieved between one and four hours after administration. The apparent differences in rates of absorption may well have been due to variation in the rate of dissolution of the gelatine capsules, as concentrations of both compounds rose in parallel in individual dogs. Concentration decay of the two compounds took place at similar rates, the ratio between SDM and Compound A being roughly 100:1.

Concentrations of SDM and Compound A were respectively predicted to be lower and higher at the site of infection, i.e. in the tissue fluid, than the concentrations in serum of Table 2, due to the respective acidic and basic properties of the compounds indicating that a closer ratio of SDM to Compound A was present in tissues than in the serum. The concentrations of both compounds still present 48 hours after dosing would be synergistically active against a broad spectrum of pathogenic bacteria. Continual effective antibacterial activity in the dog could be achieved by giving an oral dose of Compound A and SDM at 5 and 25mg/kg respectively once every 48 hours.

Comparison with Prior Art

This can be compared with results of J. D. Baggot et al, Canadian Jnl. of Comparative Medicine, 1976, 40(3), 310-317 wherein a dosage regime of 55mg/kg i/v sulphadimethoxine alone and subsequent doses at 24 hr intervals of 27.5mg/kg i/v or 55mg/kg oral suspension was recommended as a result of a study in beagle dogs.

Example 2

Bioavailability in dogs and cats of two injectable formulations of Compound A and Sulphadimethoxine

Materials and Methods

Formulations

GF = Solution in glycofurol of Compound A 2% w/v and sulphadimethoxine (SDM), 10% w/v;
OS = Suspension in Miglyol 812 of Compound A, 2% w/v and SDM, 10% w/v

Six adult beagles (three male, three female, including Dogs 1 to 4 of Example 1) and six adult male cats, housed in Coopers' kennels were in all cases given a single injection at 30mg. total a.i./kg. bodyweight, i.e. 1ml./4kg. The glycofurol solution (GF) was injected s/c in the dorsal neck region and the oily suspension (OS) was injected i/m in the quadriceps (anterior thigh) muscles. Animal weights and dose volumes are shown in Tables 3 and 4.

Blood samples (jugular venous samples or on some occasions from the cats, cephalic vein samples) were collected before and after injection at the times indicated in Tables 5 to 8).

A sample of a spontaneously voided urine was collected 24 hours after injection of one of the cats with GF.

Assay

A standard well-plate bio-assay method was used for Compound A, SDM being estimated by the Bratton-Marshall method. (The bio-assay method cannot distinguish between parent compound and any active metabolites present, but the results are expressed as mg/L Compound A).

Other Observations

The animals were observed for signs of pain during and after injection, and injection site regions were examined daily for 3 days for signs of local reactions.

Results and Discussion

None of the dogs or cats showed evidence of pain during or immediately following injection.

The results suggest that a single s/c injection of the GF solution in dogs at 30mg. total a.i./kg. should provide broad spectrum antibacterial activity for at least three days, but the same dose in cats would require daily reinforcement. The much greater concentrations of both Compound A and SDM in the single sample of feline urine obtained at 24 hours than in the corresponding serum sample indicates that for the treatment of urinary tract infection in the cat, a single injection would be effective for considerably longer than 24 hours.

Table 3.        Bodyweights of dogs and dose volumes.

| Dog | Sex | Formulation | Bodyweight (kg) | Dose (ml) |
|-----|-----|-------------|-----------------|-----------|
| 1 | F | GF | 8.5 | 2.2 |
| 2 | F | OS | 11.4 | 2.9 |
| 3 | M | GF | 18.7 | 4.7 |
| 4 | M | OS | 14.6 | 3.7 |
| 5 | M | OS | 14.3 | 3.6 |
| 6 | F | GF | 16.4 | 4.1 |

Table 4.        Bodyweights of cats and dose volumes.

| Cat | Formulation | Bodyweight (kg) | Dose (ml) |
|-----|-------------|-----------------|-----------|
| 1 | OS | 5.0 | 1.3 |
| 2 | OS | 4.0 | 1.0 |
| 3 | GF | 5.8 | 1.5 |
| 6 | GF | 5.5 | 1.4 |
| 7 | OS | 6.0 | 1.5 |
| 8 | GF | 5.6 | 1.4 |

Table 5.    Serum concentrations (mg/L) of Compound A in dogs.

|  | GF s/c | | | OS i/m | | |
|---|---|---|---|---|---|---|
| Hours p.i. | No.1 | No.6 | No.3 | No.2 | No.5 | No.4 |
| 0 | 0.03* | 0 | 0.04* | 0 | 0 | 0 |
| 1 | 0.30 | 0.13 | 0.11 | 0 | 0.08 | 0.03 |
| 4 | 0.27 | 0.24 | 0.23 | 0.05 | 0.15 | 0.09 |
| 7 | 0.40 | 0.28 | 0.29 | 0.08 | 0.20 | 0.16 |
| 24 | 0.29 | 0.28 | 0.25 | 0.16 | 0.27 | 0.24 |
| 28 | 0.24 | 0.29 | 0.29 | 0.18 | 0.26 | 0.25 |
| 31 | 0.24 | 0.28 | 0.28 | 0.20 | 0.28 | 0.27 |
| 48 | 0.18 | 0.23 | 0.21 | 0.20 | 0.20 | 0.20 |
| 55 | 0.17 | 0.19 | 0.20 | 0.17 | 0.16 | 0.16 |
| 72 | 0.09 | 0.11 | 0.10 | 0.10 | 0.08 | 0.10 |

* These two dogs had received s/c injection 4 days previously of a
solution of Compound A.

Table 6.    Serum concentrations (mg/L) of SDM in dogs.

|  | GF s/c | | | OS i/m | | |
|---|---|---|---|---|---|---|
| Hours p.i. | No.1 | No.6 | No.3 | No.2 | No.5 | No.4 |
| 0 | 0 | 0 | 0 | 0 | 0 | 0 |
| 1 | 82 | 26 | 21 | 5 | 12 | 7 |
| 4 | 95 | 79 | 90 | 23 | 43 | 26 |
| 7 | 99 | 94 | 99 | 35 | 53 | 36 |
| 24 | 47 | 56 | 51 | 34 | 42 | 38 |
| 28 | 34 | 47 | 40 | 31 | 41 | 37 |
| 31 | 30 | 45 | 30 | 29 | 43 | 35 |
| 48 | 24 | 34 | 19 | 35 | 34 | 26 |
| 55 | 17 | 21 | 12 | 27 | 25 | 19 |
| 72 | 14 | 10 | 21 | 6 | 20 | 10 |

Table 7.    Serum concentrations (mg/L) of Compound A in cats.

| Hours p.i. | GF s/c | | | OS i/m | | |
|---|---|---|---|---|---|---|
| | No.6 | No.8 | No.3 | No.7 | No.1 | No.2 |
| 0 | 0 | 0 | 0 | 0 | 0 | 0 |
| 2 | 0.44 | 0.66 | 0.54 | 0.20 | 0.05 | 0.18 |
| 7 | 0.45 | 0.66 | 0.36 | 0.22 | 0.19 | 0.24 |
| 24 | 0.06* | 0.06 | 0.05 | 0.09 | 0.11 | 0.10 |

* Urine sample from cat 6 at 24 hours contained
  10 mg/L

Table 8.    Serum concentrations (mg/L) of SDM in cats.

| Hours p.i. | GF s/c | | | OS i/m | | |
|---|---|---|---|---|---|---|
| | No.6 | No.8 | No.3 | No.7 | No.1 | No.2 |
| 0 | 0 | 0 | 0 | 0 | 0 | 0 |
| 2 | 48 | 62 | 72 | 13 | 4 | 14 |
| 7 | 41 | 51 | 35 | 16 | 14 | 16 |
| 24 | 9* | 6 | 9 | 6 | 7 | 7 |

* Urine sample from cat 6 at 24 hours contained
  205 mg/L.

## Example 3

### Comparison of bioavailability of Compound A and Trimethoprim (TMP) administered by i/v injection

### Materials and Methods

One male beagle dog, 13.5 kg and one female beagle, 11.1kg, both aged about 18 months, received i/v injections (cephalic vein) of the test compounds at 2mg base/kg, using 15mg base /ml aqueous solutions. First injections were of TMP followed after a further 4 days by Compound A. Blood sampling times are shown in Table 9.

Two young adult female cats, bodyweight around 3 kg, were given i/v injections at 2 mg/kg of TMP or Compound A, using 3 mg/base ml aqueous solutions. Blood sampling intervals are shown in Table 10.

## Results and discussion

It was found that Compound A has a greatly extended half-life in comparison with TMP in dogs. The shorter half-life in the cat (2.4 hours) compared favourably with the half-life of TMP in this species of 0.75 hours.

Compound A appeared to be less susceptible to metabolism and to be eliminated mainly by excretion.

The results indicate that Compound A may be much more suitable than TMP for the therapy of bacterial infections, particularly in domestic animals.

Table 9. Serum conc. (mg/L) in dogs of TMP and Compound A after i/v injection at 2 mg/kg.

| Hours post inj. | TMP | | Compound A | |
|---|---|---|---|---|
| | M | F | M | F |
| 0 | 0 | 0 | 0 | 0 |
| 1 | 1.10 | 1.20 | 0.24 | 0.41 |
| 3 | 0.80 | 0.74 | 0.27 | 0.34 |
| 5 | 0.40 | 0.31 | 0.26 | 0.30 |
| 7 | 0.26 | 0.21 | 0.25 | 0.29 |
| 24 | 0 | 0 | 0.11 | 0.124 |
| 30 | 0 | 0 | NO SAMPLE | 0.10 |
| 48 | 0 | 0 | 0.039 | 0.046 |
| Est. $t_{1/2}$ (h) | 2.6 hours | | | 15 hours |

Table 10. Serum conc. (mg/L) in cats of TMP and Compound A after i/v injection at 2 mg/kg.

| Hours post inj. | TMP | | Compound A | |
|---|---|---|---|---|
| | 1 | 2 | 3 | 4 |
| 0 | 0 | 0 | 0 | 0 |
| 1 | 0.46 | 0.44 | 0.32 | 0.31 |
| 3 | 0.06 | 0.084 | 0.16 | 0.14 |
| 5 | 0 | 0 | 0.10 | 0.086 |
| 7 | 0 | 0 | 0.072 | NO SAMPLE |
| 24 | 0 | 0 | 0 | 0 |
| Est. $t_{1/2}$ (h) | 0.75 hours | | 2.4 hours | |

## Example 4

## Drench

Here the suspended solids content, and hence the viscosity, of the finished preparation is reduced by taking the sulphonamide into solution as its sodium salt. The salt is produced in situ by the addition of sodium hydroxide.

Optionally, methyl cellulose may be incorporated to increase the viscosity of the finished product and maintain compound A in uniform suspension.

|  | % w/v |
| --- | --- |
| Compound A (in fine powder form) | 2.0g |
| Sulphadimethoxine | 10.0g |
| Sodium Hydroxide | 1.3g or |
|  | qs to produce pH 10-11 |
| Polysorbate 80 (wetter) | 0.1g |
| Purified Water | to 100.0 ml |

The Sulphadimethoxine was suspended in approximately 50 ml water and 30% sodium hydroxide solution added. The pH of the resulting solution was adjusted to approximately 11.0 with further Sodium Hydroxide solution.

Compound A was dispersed in a solution of the Polysorbate 80 in approximately 20 ml Water. This was added to the solution of Sulphadimethoxine Sodium and the volume made up to 100ml with Purified Water.

Example 5

Intramuscular Injection

| Compound A (in fine powder form) | 2.0g |
| --- | --- |
| Sulphadimethoxine(in fine powder form) | 10.0g |
| Fractionated Coconut Oil | to 100.ml |

The Compound A and Sulphadimethoxine were sterilised by heating at 160°C for 1 hour. Under aseptic conditions the drugs were added to the carrier, previously sterilised by filtration through a membrane filter.

The suspension was milled aseptically to disperse aggregates, and filled aseptically into vials and sealed

In this presentation both drugs remained in suspension in the vehicle.

Example 6

Oral Paste

| Compound A | 4.0g |
| --- | --- |
| Sulphadimethoxine | 20.0g |
| Xanthan Gum | 1.0g |
| Methyl Hydroxybenzoate | 0.1g |
| Polysorbate 80 | 0.2g |
| Colloidal Silicon Dioxide | 5.0g |
| Purified Water | to 100.0 g |

Polysorbate 80 and Methyl Hydroxybenzoate were dissolved in the bulk of the water. Xanthan Gum was added and dispersed and allowed to swell. Compound A and Sulphadimethoxine were added and dispersed followed by the Colloidal Silicon Dioxide.

The rationale for this formulation is similar to that for the Oral Drench but both drugs remained in suspension. High viscosity is a feature of the product. Xanthan Gum contributes to thickening the product as does the Colloidal Silicon Dioxide..

Example 7

Ointment

| | |
|---|---|
| Compound A | 2.0g |
| Sulphadimethoxine | 10.0g |
| White Soft Paraffin | 88.0g |

White Soft Paraffin was melted at 60°C. Compound A and Sulphadimethoxine were added and dispersed and allowed to cool. The product was filled into collapsible metal tubes.

Example 8

Injectable solution

Each 100ml contains:

| | |
|---|---|
| Compound A | 2.0g |
| Sulphadimethoxine | 10.0g |
| Tetraglycol (Agrar) | to 100 ml |

The components were mixed together in the cold until dissolved, then sterilised by filtration. The resulting solution was filled into ampoules or glass vials, and seated under nitrogen. The vials were then autoclaved.

Example 9

Tablet for Dogs

Each tablet contains:

| | |
|---|---|
| Compund A | 100mg |
| Sulphadimethoxine | 500mg |
| Sodium Starch Glycollate (Explotab) | 50mg |
| Microcrystalline Cellulose (Avicel)* | 307.5mg |
| Polyvinylpyrrolidone (Povidone K30) | 40.mg |
| Magnesium Stearate | 2.5mg |
| | ———— |
| | 1000mg |

The first four ingredients were mixed together and granulated with a solution of the polyvinylpyrrolidone in 50% aqueous alcohol. The resultant granules were dried, blended with magnesium stearate and compressed on a Manesty rotary tabletting machine to give tablets containing 600mg total drugs.

## Example 10

### Tablet for Cats and small dogs

Each tablet contains:

| | |
|---|---|
| Compound A | 10mg |
| Sulphadimethoxine | 50mg |
| Sodium Starch Glycollate (Explotab) | 5mg |
| Microcrystalline Cellulose (Avicel)* | 30.75mg |
| Polyvinylpyrrolidone (Povidone K30) | 4mg |
| Magnesium Stearate | 0.25mg |
| | ———— |
| | 100mg |

* Registered Trade Mark

The first four ingredients were mixed together and granulated with a solution of the polyvinylpyrrolidone in 50% aqueous alcohol. The resultant granules were dried, blended with magnesium stearate and compressed on a Manesty rotary tabletting machine to give tablets containing 60mg total drugs. The tablets were then (optionally) coated using the following:

### Coating

The coating, comprising, in quantities per tablet,
Methocel E5 (6.4mg) and Methocel* E15 (1.8mg) : Polyethylene glycol (1.8mg)
was obtained premixed (Colorcon) or was mixed in the above proportions and then made up into aqueous solution, applied to the tablets and the water evaporated to give a coating of 10% of tablet weight.
In general, the tablets are coated when used for cats and dogs and not coated when used for dogs alone.

## Claims

### Claims for the following Contracting States : AT, BE, CH, DE, DK, FR, GB, IT, LI, LU, NL, SE

1. A composition comprising the compounds 2,4-diamino-5-(8-dimethyl amino-7-methyl-5-quinolylmethyl)pyrimidine and sulphadimethoxine or salts thereof.

2. A composition according to claim 1 wherein the said compounds are present in a ratio of between 1:1 and 1:20.

3. A composition according to claim 1 wherein the compounds are present in a ratio of 1:5.

4. A composition according to any of claims 1 to 3 for parenteral administration.

5. A composition according to claim 1 for oral administration wherein the compounds are present in the ratio of between 1:4 and 1:10.

6. A composition according to claim 1 for oral administration wherein the compounds are present in the ratio of 1:5.

7. A composition according to any one of claims 1 to 6 for use in the treatment of bacterial infections in an-

*Registered Trade Mark

imals.

**8.** A composition according to claim 1 comprising a tablet which contains from 2 to 20 mg of 2,4-diamino-5-(8-dimethylamino-7-methyl-5-quinolylmethyl)pyrimidine and from 10 to 100 mg of sulphadimethoxine.

**9.** A composition according to claim 1 comprising a tablet which contains from 10 to 200 mg of 2,4-diamino-5-(8-dimethylamino-7-methyl-5-quinolylmethyl)pyrimidine and from 50 to 1000mg of sulphadimethoxine.

**10.** A composition which comprises the compounds 2,4-diamino-5-(8-dimethylamino-7-methyl-5-quinolylmethyl)pyrimidine and sulphadimethoxine for use in the manufacture of a medicament.

**Claims for the following Contracting States : ES, GR**

**1.** A method for the preparation of a composition comprising the compounds 2,4-diamino-5-(8-dimethylamino-7-methyl-5-quinolylmethyl)pyrimidine and sulphadimethoxine, or salts thereof, which method comprises bringing into association the 2,4-diamino-5-(8-dimethylamino-7-methyl-5-quinolylmethyl)pyrimidine and the sulphadimethoxine, and optionally a pharmaceutically acceptable carrier therefor.

**2.** A method according to claim 1 wherein the said compounds are present in a ratio of between 1:1 and 1:20.

**3.** A method according to claim 1 wherein the compounds are present in the ratio of 1:5.

**4.** A method according to claim 1 to 3 wherein the composition is for parenteral administration.

**5.** A method according to claim 1 wherein the composition is for oral administration and the compounds are present in the ratio of between 1:4 and 1:10.

**6.** A method according to claim 1 to 5 wherein the composition is for the treatment of bacterial infections in animals.

**7.** A method according to claim 6 wherein the composition is for the treatment of bacterial infections in animals by oral administration.

**8.** A method according to claim 6 wherein the composition is a tablet which contains from 2 to 20 mg of 2,4-diamino-5-(8-dimethylamino-7-methyl-5-quinolylmethyl)pyrimidine and from 10 to 100 mg of sulphadimethoxine.

**9.** A method according to claim 1 wherein the composition is a tablet which contains from 10 to 200 mg of 2,4-diamino-5-(8-dimethylamino-7- methyl-5-quinolylmethyl)pyrimidine and from 50 to 1000mg of sulphadimethoxine.

**10.** A composition which comprises the compounds 2,4-diamino-5-(8-dimethylamino-7-methyl-5-quinolylmethyl)pyrimidine and sulphadimethoxine for use in the manufacture of a medicament.

**Patentansprüche**

**Patentansprüche für folgende Vertragsstaaten : AT, BE, DR, CH, DE, DK, GB, IT, LI, LU, NL, SE**

**1.** Zusammensetzung, die die Verbindungen 2,4-Diamino-5-(8-dimethylamino-7-methyl-5-chinolylmethyl)-pyrimidin und Sulphadimethoxin oder Salze davon enthält.

**2.** Zusammensetzung nach Anspruch 1, dadurch gekennzeichnet, daß die Verbindungen in einem Verhältnis zwischen 1:1 und 1:20 vorhanden sind.

**3.** Zusammensetzung nach Anspruch 1, dadurch gekennzeichnet, daß die Verbindungen in einem Verhältnis von 1:5 vorhanden sind.

**4.** Zusammensetzung nach einem der Ansprüche 1 bis 3 zur parenteralen Verabreichung.

**5.** Zusammensetzung nach Anspruch 1 für die orale Verabreichung, dadurch gekennzeichnet, daß die Verbindungen in dem Verhältnis zwischen 1:4 und 1:10 vorhanden sind.

**6.** Zusammensetzung nach Anspruch 1 für die orale Verabreichung, dadurch gekennzeichnet, daß die Verbindungen in dem Verhältnis von 1:5 vorhanden sind.

**7.** Zusammensetzung nach einem der Ansprüche 1 bis 6 zur Verwendung bei der Behandlung von bakteriellen Infektionen bei Tieren.

**8.** Zusammensetzung nach Anspruch 1, enthalten in einer Tablette, welche 2 bis 20 mg 2,4-Diamino-5-(8-dimethylamino-7-methyl-5-chinolylmethyl)-pyrimidin und 10 bis 100 mg Sulphadimethoxin enthält.

**9.** Zusammensetzung nach Anspruch 1, enthalten in einer Tablette, welche 10 bis 200 mg 2,4-Diamino-5-(8-dimethylamino-7-methyl-5-chinolylmethyl)-pyrimidin und 50 bis 1000 mg Sulphadimethoxin enthält.

**10.** Zusammensetzung, die die Verbindungen 2,4-Diamino-5-(8-dimethylamino-7-methyl-5-chinolylmethyl)-pyrimidin und Sulphadimethoxin zur Verwendung bei der Herstellung eines Arzneimittels enthält.

**Patentansprüche für folgende Vertragsstaaten : ES, GR**

**1.** Verfahren zur Herstellung einer Zusammensetzung, die die Verbindungen 2,4-Diamino-5-(8-dimethylamino-7-methyl-5-chinolylmethyl)-pyrimidin und Sulphadimethoxin oder Salze davon enthält, dadurch gekennzeichnet, daß das 2,4-Diamino-5-(8-dimethylamino-7-methyl-5-chinolylmethyl)-pyrimidin und das Sulphadimethoxin und wahlweise ein pharmazeutisch annehmbarer Träger dafür zur Assoziation gebracht werden.

**2.** Verfahren nach Anspruch 1, dadurch gekennzeichnet, daß die Verbindungen in einem Verhältnis zwischen 1:1 und 1:20 vorhanden sind.

**3.** Verfahren nach Anspruch 1, dadurch gekennzeichnet, daß die Verbindungen in einem Verhältnis von 1:5 vorhanden sind.

**4.** Verfahren nach Anspruch 1 bis 3, dadurch gekennzeichnet, daß die Zusammensetzung zur parenteralen Verabreichung dient.

**5.** Verfahren nach Anspruch 1, dadurch gekennzeichnet, daß die Zusammensetzung zur oralen Verabreichung dient und die Verbindungen in dem Verhältnis zwischen 1:4 und 1:10 vorhanden sind.

**6.** Verfahren nach Anspruch 1 bis 5, dadurch gekennzeichnet, daß die Zusammensetzung zur Behandlung von bakteriellen Infektionen bei Tieren dient.

**7.** Verfahren nach Anspruch 6, dadurch gekennzeichnet, daß die Zusammensetzung zur Behandlung von bakteriellen Infektionen bei Tieren durch orale Verabreichung dient.

**8.** Verfahren nach Anspruch 6, dadurch gekennzeichnet, daß die Zusammensetzung eine Tablette ist, welche 2 bis 20 mg 2,4-Diamino-5-(8-dimethylamino-7-methyl-5-chinolylmethyl)-pyrimidin und 10 bis 100 mg Sulphadimethoxin enthält.

**9.** Verfahren nach Anspruch 1, dadurch gekennzeichnet, daß die Zusammensetzung eine Tablette ist, welche 10 bis 200 mg 2,4-Diamino-5-(8-dimethylamino-7-methyl-5-chinolylmethyl)-pyrimidin und 50 bis 1000 mg Sulphadimethoxin enthält.

**10.** Zusammensetzung, die die Verbindungen 2,4-Diamino-5-(8-dimethylamino-7-methyl-5-chinolylmethyl)-pyrimidin und Sulphadimethoxin zur Verwendung bei der Herstellung eines Arzneimittels enthält.

EP 0 384 722 B1

**Revendications**

**Revendications pour les Etats contractants suivants : AT, BE, CH, DE, DK, FR, GB, IT, LI, LU, NL, SE**

1. Composition comprenant les composés 2,4-diamino-5-(8-diméthylamino-7-méthyl-5-quinoléylméthyl)pyrimidine et sulfadiméthoxine, ou leurs sels.

2. Composition selon la revendication 1, dans laquelle lesdits composés sont présents dans une proportion comprise entre 1:1 et 1:20.

3. Composition selon la revendication 1, dans laquelle les composés sont présents dans une proportion de 1:5.

4. Composition selon l'une quelconque des revendications 1 à 3, destinée à l'administration parentérale.

5. Composition selon la revendication 1 destinée à l'administration orale, dans laquelle les composés sont présents dans une proportion comprise entre 1:4 et 1:10.

6. Composition selon la revendication 1 destinée à l'administration orale, dans laquelle les composés sont présents dans une proportion de 1:5.

7. Composition selon l'une quelconque des revendications 1 à 6, utilisable dans le traitement d'infections bactériennes chez des animaux.

8. Composition selon la revendication 1, comprenant un comprimé qui contient de 2 à 20 mg de 2,4-diamino-5-(8-diméthylamino-7-méthyl-5-quinoléylméthyl)pyrimidine et de 10 à 100 mg de sulfadiméthoxine.

9. Composition selon la revendication 1, comprenant un comprimé qui contient de 10 à 200 mg de 2,4-diamino-5-(8-diméthylamino-7-méthyl-5-quinoléylméthyl)pyrimidine et de 50 à 1000 mg de sulfadiméthoxine.

10. Composition qui comprend les composés 2,4-diamino-5-(8-diméthylamino-7-méthyl-5-quinoléylméthyl)pyrimidine et sulfadiméthoxine à utiliser dans la fabrication d'un médicament.

**Revendications pour les Etats contractants suivants : ES, GR**

1. Procédé de préparation d'une composition comprenant les composés 2,4-diamino-5-(8-diméthylamino-7-méthyl-5-quinoléylméthyl)pyrimidine et sulfadiméthoxine, ou leurs sels, ce procédé comprenant l'association de la 2,4-diamino-5-(8-diméthylamino-7-méthyl-5-quinoléylméthyl)pyrimidine et de la sulfadiméthoxine, et éventuellement d'un véhicule pharmaceutiquement acceptable pour ces composés.

2. Procédé selon la revendication 1, dans lequel lesdits composés sont présents dans une proportion comprise entre 1:1 et 1:20.

3. Procédé selon la revendication 1, dans lequel les composés sont présents dans une proportion de 1:5.

4. Procédé selon les revendications 1 à 3, dans lequel la composition est destinée à l'administration parentérale.

5. Procédé selon la revendication 1, dans lequel la composition est destinée à l'administration orale et les composés sont présents dans une proportion comprise entre 1:4 et 1:10.

6. Procédé selon les revendications 1 à 5, dans lequel la composition est faite pour traiter les infections bactériennes chez les animaux.

7. Procédé selon la revendication 6, dans lequel la composition est faite pour traiter les infections bactériennes chez les animaux par administration orale.

8. Procédé selon la revendication 6, dans lequel la composition est un comprimé qui contient de 2 à 20 mg

15

de 2,4-diamino-5-(8-diméthylamino-7-méthyl-5-quinoléylméthyl)pyrimidine et de 10 à 100 mg de sulfadiméthoxine.

9. Procédé selon la revendication 1, dans lequel la composition est un comprimé qui contient de 10 à 200 mg de 2,4-diamino-5-(8-diméthylamino-7-méthyl-5-quinoléylméthyl)pyrimidine et de 50 à 1000 mg de sulfadiméthoxine.

10. Composition qui comprend les composés 2,4-diamino-5-(8-diméthylamino-7-méthyl-5-quinoléylméthyl)pyrimidine et sulfadiméthoxine à utiliser dans la fabrication d'un médicament.